# FASCICULE DE BREVET EUROPEEN

(11) **EP 2 861 276 B1**
(45) Date de publication et mention de la délivrance du brevet: **05.12.2018**
(21) Numéro de dépôt: 13728211.7
(22) Date de dépôt: 13.06.2013
(51) Int. Cl.: A61M 39/00, A61M 5/00, A61M 39/24, A61M 5/148

(54) **ENSEMBLE D'INJECTION DE PRODUIT LIQUIDE ET VISQUEUX**
ANORDNUNG ZUR INJEKTION EINES ZÄHFLÜSSIGEN PRODUKTS
ASSEMBLY FOR INJECTING A VISCOUS LIQUID PRODUCT

(30) Priorité: 13.06.2012 FR 1255530; 13.06.2012 US 201261659288 P
(43) Date de publication de la demande: 22.04.2015
(73) Titulaire: Medex, 69800 Saint-Priest (FR)
(72) Inventeur: TERRASSE, Samuel, F-38080 Saint Alban De Roche (FR); MATRAY, Damien, F-38300 Bourgoin-jallieu (FR); MANEUF, Nathalie, 69008 Lyon (FR); MILCENT, Anne-Marie, F-69001 Lyon (FR)
(74) Mandataire: Regimbeau
(86) Numéro de dépôt international: PCT/EP2013/062238
(87) Numéro de publication internationale: WO 2013/186300

(56) Documents cités:
- EP-A1- 0 648 513
- EP-A1- 0 650 739
- WO-A2-99/21600
- FR-A1- 2 850 027
- US-A- 3 888 239
- US-A- 4 596 575
- US-A- 5 078 683
- US-A- 5 165 874
- US-A1- 2011 301 539

## Description

### DOMAINE TECHNIQUE

La présente invention concerne le domaine technique général de l'administration de produit, notamment liquide ou visqueux, à un patient, par exemple par voie parentérale, éventuellement à partir d'une poche souple.

Plus précisément, la présente invention concerne un ensemble à usage médical permettant l'injection de produit liquide ou visqueux dans le corps humain.

### PRESENTATION GENERALE DE L'ART ANTERIEUR

Les développements de la médecine ont conduit à mettre au point différents procédés d'analyse et de contrôle de l'état des patients.

Parmi ces procédés, il existe les analyses faites après injection d'un produit de contraste, par exemple pour l'imagerie médicale qui regroupe aussi bien l'imagerie par rayons X que l'Imagerie par Résonance Magnétique (IRM) ou la médecine nucléaire.

Dans la majorité des cas, l'opération d'injection de produit de contraste nécessite le transfert du produit de contraste entre un récipient contenant ce produit de contraste et le patient. Ce transfert est mis en oeuvre en utilisant un ensemble à usage médical comportant une ligne d'injection - telle qu'un tube connecté au récipient d'une part et au patient d'autre part - pour le transfert du produit de contraste entre le récipient et le patient.

Il est souvent nécessaire de remplir le récipient de produit de contraste préalablement au transfert de celui-ci vers le patient. Dans ce cas, l'opération d'injection comprend :
- une étape de remplissage du récipient avec le produit de contraste à partir de la source d'alimentation, et
- une étape de transfert du produit de contraste entre le récipient et le patient.

Pour remplir le récipient, on utilise par exemple un dispositif autonome permettant de remplir le récipient à partir d'une source d'alimentation.

Toutefois une telle solution présente l'inconvénient de nécessiter un grand nombre de manipulations pour :
- la connexion/déconnexion du récipient au dispositif autonome d'une part, et
- la connexion/déconnexion du récipient à la ligne d'injection d'autre part.
Ces manipulations peuvent réduire la qualité de l'asepsie.

Pour remédier à cet inconvénient, EP 0 648 513 propose un ensemble à usage médical comportant :
- une ligne d'injection pour le transfert du produit à injecter entre le récipient et le patient, et
- une ligne de remplissage pour le transfert du produit à injecter entre une source d'alimentation et le récipient.

Cet ensemble présente l'avantage de limiter le nombre de connexions/déconnexions nécessaires à la mise en oeuvre de l'étape de remplissage du récipient.

En utilisant l'ensemble à usage médical décrit dans EP 0 648 513, l'étape de remplissage peut être mise en oeuvre selon trois techniques différentes :
i) un remplissage par gravité de la source d'alimentation:
   Un inconvénient de cette technique est que la durée de remplissage du récipient est très longue, ce qui induit une durée longue de l'opération d'injection. Les deux techniques de remplissage suivantes ont pour but de réduire la durée de remplissage :
ii) un remplissage par aspiration du produit contenu dans la source d'alimentation :
   Un inconvénient de cette technique est qu'elle peut dégrader la qualité du produit de contraste. En effet, lors d'un remplissage par aspiration notamment à l'aide d'une seringue, une dépression importante est créée dans la ligne de remplissage et l'eau composant le produit de contraste peut passer en phase gazeuse. Ce phénomène est amplifié dans le cas où le produit de contraste est chauffé.
   Un autre inconvénient de cette technique concerne les pertes de charges dans la ligne de remplissage. En effet, le déplacement du produit de contraste étant induit par la dépression créée par la seringue, la vitesse de déplacement du produit de contraste est limitée par la différence de pression générée entre la pression atmosphérique et la pression dans la seringue, soit une limite physique d'un bar ;
iii) un remplissage par mise en pression de la source d'alimentation :
   Un inconvénient de cette technique est qu'elle ne peut être mise en oeuvre qu'avec une source d'alimentation rigide. Par ailleurs, cette technique ne peut être mise en oeuvre qu'à une pression très faible (inférieure à un bar) afin de limiter les risques de fuite. Enfin, cette technique ne permet pas de déterminer la quantité de produit transféré entre la source primaire et le récipient.

US 2011/301539, US 4 596 575, US 3 888 239, FR 2 850 027, EP 0 650 739, WO 99/21600, US 5 165 874 et US 5 078 683 proposent d'autres ensembles à usage médical.

Un but de la présente invention est de proposer un ensemble à usage médical permettant de pallier au moins l'un des inconvénients précités.

### PRESENTATION DE L'INVENTION

A cet effet, l'invention propose un ensemble à usage médical pour l'injection à un patient d'un produit liquide ou visqueux, tel qu'un produit de contraste, caractérisé en ce que l'ensemble comporte :
- une ligne d'injection pour le transfert du produit à injecter entre un récipient et le patient, ladite ligne d'injection comportant une partie tubulaire amont, une partie tubulaire aval et un clapet anti-retour à seuil sur la partie tubulaire aval, ledit clapet anti-retour à seuil permettant le passage du produit liquide depuis le récipient vers le patient,
- une ligne de remplissage pour le transfert du produit à injecter entre une source d'alimentation et le récipient par l'intermédiaire de la partie tubulaire amont, ladite ligne de remplissage étant raccordée à la ligne d'injection entre les parties tubulaires amont et aval et comprenant un clapet anti-retour permettant le passage du produit liquide depuis la source d'alimentation vers le récipient,
- une interface de couplage sur la ligne de remplissage destinée à être couplée à une unité de pompage permettant le déplacement du liquide entre la source d'alimentation et le récipient.

Des aspects préférés mais non limitatifs de l'ensemble à usage médical selon l'invention sont les suivants :
- la ligne de remplissage comprend une tubulure en amont de l'interface de couplage et une tubulure en aval de l'interface de couplage, la dureté de la tubulure en amont de l'interface de couplage étant supérieure à la dureté de la tubulure en aval de l'interface de couplage ;
- la dureté de la tubulure en amont de l'interface de couplage est égale ou supérieure à 70 ShA (la dureté Shore, exprimée en Shore A ou ShA, correspond à une unité de mesure de la dureté d'élastomères ou de certaines matières plastiques très bien connue de l'homme du métier et reconnue par les normes internationales ISO 868 et 7619, ASTM D 2240 et DIN 53505) ;
- la ligne de remplissage comprend une tubulure en amont de l'interface de couplage et une tubulure en aval de l'interface de couplage, le diamètre intérieur de la tubulure en amont de l'interface de couplage étant supérieur au diamètre intérieur la partie tubulaire aval de la ligne d'injection ;
- le diamètre intérieur de la tubulure en amont de l'interface de couplage est compris entre 3 et 6 millimètres ;
- la ligne de remplissage comprend une tubulure en amont de l'interface de couplage et une tubulure en aval de l'interface de couplage, la dureté de la tubulure en aval de l'unité de pompage étant inférieure à la dureté de la partie tubulaire aval de la ligne d'injection ;
- la dureté shore de la tubulure en aval de l'unité de pompage est comprise entre 60 et 80 ShA ;
- la ligne de remplissage comprend une tubulure en amont de l'interface de couplage et une tubulure en aval de l'interface de couplage, le diamètre de la tubulure en aval de l'interface de couplage étant supérieur au diamètre de la partie tubulaire aval de la ligne d'injection ;
- l'ensemble à usage médical comprend en outre le récipient, ledit récipient étant connecté à la ligne d'injection préalablement à son utilisation ;
- le récipient est une poche composée de deux feuilles de matériau superposées et soudées à leur périphérie pour définir un espace destiné à contenir le produit liquide ou visqueux ;
- l'ensemble à usage médical comprend en outre une connexion inamovible entre le récipient et la ligne d'injection.

Le lecteur appréciera que l'ensemble à usage médical peut être utilisé dans un injecteur du type décrit dans la demande de brevet français n° FR 1255530 déposée au nom de la société MEDEX. Dans ce cas, le récipient de l'ensemble à usage médical est une poche comportant des feuillets superposés et soudés à leur périphérie et une conduite pour le passage du produit liquide ou visqueux. Cette poche est destinée à être positionnée dans l'injecteur de sorte que la conduite de la poche constitue l'élément de la poche le plus éloigné du sol, pour qu'un éventuel volume d'air contenu dans la poche soit en contact avec la conduite lorsque la poche est remplie de produit liquide ou visqueux.

### PRESENTATION DES FIGURES

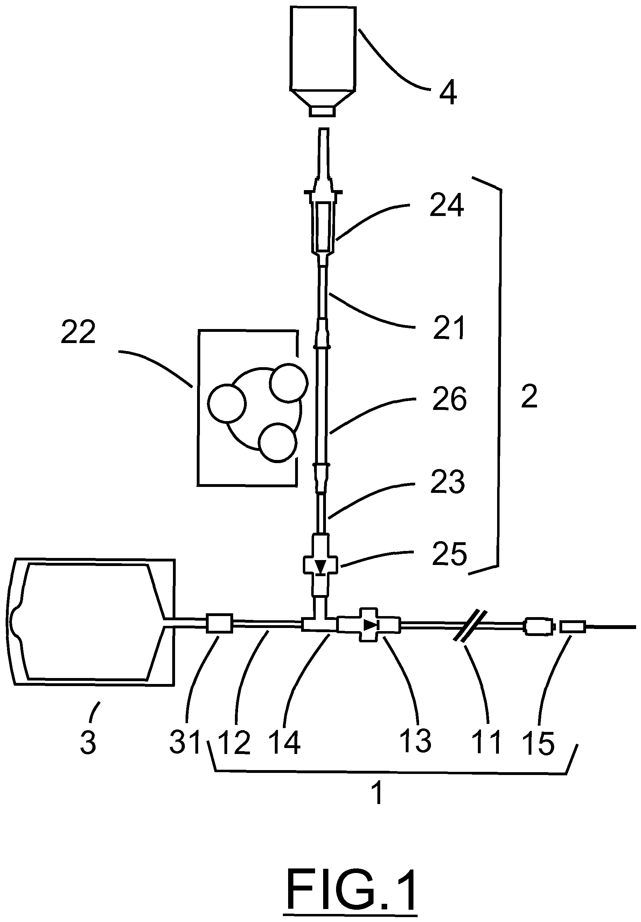

D'autres caractéristiques et avantages de l'invention ressortiront encore de la description qui suit, laquelle est purement illustrative et non limitative et doit être lue en regard de la figure 1 qui illustre un mode de réalisation d'un ensemble à usage médical pour l'injection d'un produit liquide ou visqueux à un patient.

### DESCRIPTION DE MODES DE REALISATION DE L'INVENTION

Comme indiqué précédemment, une opération d'injection comprend généralement deux phases :
- une phase de remplissage d'un récipient avec un produit à injecter, et
- une phase d'administration du produit au patient à partir du récipient.

En effet, le récipient utilisé lors d'une opération d'injection est généralement vide pour permettre à l'utilisateur d'utiliser celui-ci avec différents types et/ou concentrations de produits à injecter.

L'ensemble d'injection à usage médical décrit ci-après permet la mise en oeuvre de ces deux phases (i.e. remplissage/administration) lors d'une opération d'injection.

### Généralités

En référence à la figure 1, on a illustré un mode de réalisation de l'ensemble à usage médical pour l'injection à un patient d'un produit liquide ou visqueux, tel qu'un produit de contraste.

L'ensemble à usage médical comprend une ligne d'injection 1 et une ligne de remplissage 2. La ligne d'injection 1 permet le transfert du produit à injecter entre un récipient 3 et le patient. La ligne de remplissage 2 permet le transfert du produit à injecter entre une source d'alimentation primaire 4 et le récipient 3.

La présence d'une ligne de remplissage 2 permet d'améliorer l'asepsie de l'opération d'injection, le nombre de connexions/déconnexions nécessaires pour permettre le remplissage du récipient 3 étant limité.

L'ensemble à usage médical comprend également une interface de couplage 26 sur la ligne de remplissage 2. Cette interface de couplage 26 permet de coupler une unité de pompage 22 à la ligne de remplissage de l'ensemble à usage médical.

La présence d'une interface de couplage 26 lorsqu'elle est associée à une unité de pompage 22 permet d'accélérer la vitesse de remplissage tout en limitant les risques de dégradation de la qualité du produit liquide déplacé entre la source d'alimentation primaire 4 et le récipient 3.

L'unité de pompage 22 peut être une pompe péristaltique - composée de galets et d'un moteur - et l'interface de couplage 26 peut être un tuyau souple destiné à venir en contact avec les galets de la pompe péristaltique. Le diamètre, l'épaisseur et l'élasticité du tuyau souple sont adaptés pour permettre d'améliorer le couplage de la ligne de remplissage 2 avec l'unité de pompage 22. En particulier, l'interface de pompage peut être un tuyau:
- de diamètre intérieur compris entre 2 et 8 millimètres, préférentellement compris entre 3 et 6 millimètres et plus préférentiellement égal à 4,8 millimètres,
- d'épaisseur comprise entre 1 et 3 millimètres, préférentiellement comprise entre 1 et 2 millimètres et plus préférentiellement égale à 1,6 millimètres, et
- de dureté Shore A comprise entre 55 et 75 ShA, préférentiellement comprise entre 55 et 65 ShA et plus préférentiellement égale à 60 ShA.

La présence d'une interface de couplage 26 intégrée à la ligne de remplissage 2 permet d'éviter une mise en contact direct de l'unité de pompage 22 avec la solution injectable.

Ainsi, il n'est plus nécessaire de nettoyer l'unité de pompage 22 à chaque remplacement de la source d'alimentation, et notamment lorsque les sources d'alimentation successivement utilisées contiennent des produits de natures différentes.

On améliore également l'asepsie de l'opération de remplissage, puisque l'unité de pompage 22 n'est pas directement connectée à l'ensemble à usage médical, mais est couplée à celui-ci par l'intermédiaire de l'interface de couplage 26.

### Ligne d'injection

La ligne d'injection 1 comporte une partie tubulaire amont 12, une partie tubulaire aval 11 et un connecteur 14 à trois entrées - notamment de type connecteur en Y ou T - entre les parties tubulaires amont et aval 11, 12. Le connecteur 14 à trois entrées permet de relier la ligne de remplissage 2 à la ligne d'injection 1.

L'une des extrémités de la ligne d'injection 1 comprend un élément de raccordement - tel qu'un *« luer lock »* - destiné à être connecté à un conduit d'injection 15. Ce conduit d'injection 15 est, par exemple, un cathéter, une aiguille hypodermique ou intraveineuse ou tout autre type de conduit d'injection connu de l'homme du métier.

L'autre extrémité de la ligne d'injection 1 est connectée au récipient 3 destiné à contenir le produit à injecter au patient.

Avantageusement, le récipient 3 peut être intégré à l'ensemble à usage médical. En d'autres termes, l'ensemble à usage médical comprend le récipient 3. Dans ce cas, le récipient 3 est pré-connecté à la ligne d'injection 1 lors de la fabrication de l'ensemble à usage médical.

Ainsi, l'utilisateur n'a pas besoin de connecter le récipient 3 à la ligne d'injection 1 lors de l'utilisation de l'ensemble à usage médical.

Ceci permet de limiter le nombre de manipulations nécessaires à l'installation de l'ensemble à usage médical. Ceci permet également d'augmenter l'asepsie de l'ensemble à usage médical. Enfin, ceci permet de limiter les risques d'erreur - par exemple liés à la connexion d'un récipient non adapté à l'ensemble à usage médical - lors de l'installation de l'ensemble à usage médical.

De préférence, l'ensemble à usage médical comprend en outre une connexion inamovible 31 entre le récipient 3 et la ligne d'injection 1. Ceci permet de limiter les risques de déconnexion entre le récipient 3 et la ligne d'injection 1.

On entend, dans le cadre de la présente invention, par « *connexion inamovible »* un élément (par exemple une soudure, un collage, ou tout autre élément connu de l'homme du métier) permettant un assemblage permanent de deux pièces de sorte que ces deux pièces ne puissent plus être séparées l'une de l'autre, l'assemblage des deux pièces par cet élément étant réalisé préalablement à l'utilisation de l'ensemble, notamment lors de la fabrication de l'ensemble.

Dans tous les cas, le récipient 3 destiné à contenir le produit à injecter peut être :
- une seringue composée d'un corps cylindrique dans lequel est logé un piston recouvert d'un joint élastomère sur sa face avant, ledit piston étant apte à se déplacer en translation entre une position rétractée (où la seringue est vide) et une position déployée (où la seringue est remplie de produit à injecter),
- une poche souple composée par exemple de deux feuilles superposées et soudées à leur périphérie pour définir un espace destiné à contenir le produit liquide ou visqueux,
- ou tout autre type de récipient connu de l'homme du métier.

Toutefois dans certains modes de réalisation, on privilégiera un récipient 3 de type poche, notamment pour améliorer l'asepsie de l'ensemble à usage médical. En effet, dans le cas d'un récipient 3 de type seringue, la portion interne de corps cylindrique située à l'arrière du joint élastomère est en contact avec l'air ambiant, ce qui, lors de déplacements du piston entre les positions déployée et rétractée, peut diminuer l'asepsie.

La ligne d'injection 1 peut également comprendre un clapet anti-retour 13 sur la partie tubulaire aval 11. Ce clapet anti-retour 13 permet le passage de liquide dans un sens unique, à savoir du récipient 3 vers le patient.

Avantageusement, le clapet anti-retour 13 peut présenter un seuil d'ouverture élevé (i.e. supérieur à 500 mbar). Ceci permet d'éviter les risques de transfert de produit liquide directement vers le patient (sans passer par le récipient) lors de la phase de remplissage.

Le clapet anti-retour 13 peut également présenter un seuil d'ouverture plus élevé, notamment supérieur à 1,5 bar (idéalement supérieur ou égal à 2 bars) pour permettre l'utilisation de l'ensemble à usage médical dans un injecteur du type décrit dans la demande de brevet français n° FR 1255530 au nom de la société MEDEX.

Un tel injecteur comprend un boîtier constitué de deux demi-coques articulées afin de permettre le déplacement relatif des demi-coques l'une par rapport à l'autre entre :
- une position ouverte pour la mise en place du récipient 3 de l'ensemble à usage médical, et
- une position fermée pour la mise en oeuvre des phases de remplissage et d'administration.

L'une des demi-coques comprend une vessie - dite « vessie active » - déformable à volume variable sous l'action d'une source de puissance hydraulique alimentant ladite vessie en fluide hydraulique. L'autre des demi-coques comprend un coussin déformable à volume constant - dit « coussin passif ».

Le fait que le seuil d'ouverture de la valve à seuil soit supérieur à 1,5 bar (et de préférence supérieur ou égal à 2 bars) permet de garantir un bon plaquage du récipient contre la vessie et le coussin. Ce placage est nécessaire pour permettre à un tel injecteur de contrôler :
- la quantité de produit de contraste injectée au patient lors d'une phase d'administration,
- la quantité de produit de contraste introduite dans le récipient lors d'une phase de remplissage.

En effet, lors d'une phase d'administration, le principe de fonctionnement d'un tel injecteur est le suivant : la vessie est remplie de fluide hydraulique de sorte que les parois du récipient soient plaquées contre les parois du coussin et de la vessie. Lorsque les parois de la vessie et du coussin sont plaquées contre le récipient, l'introduction d'une quantité donnée de fluide hydraulique à l'intérieur de la vessie induit l'expulsion de la même quantité de produit de contraste hors du récipient. Par exemple, un millilitre de fluide incompressible introduit dans la vessie « chasse » mécaniquement un millilitre de produit de contraste hors du récipient 3. Ainsi, une fois les parois du récipient plaquées contre la vessie et le coussin, il est possible de contrôler avec précision la quantité de produit injectée au patient en mesurant la quantité de fluide hydraulique introduit dans la vessie.

Lors d'une phase de remplissage, le principe de fonctionnement d'un tel injecteur est le suivant : la vessie est remplie de fluide hydraulique de sorte que les parois du récipient soient plaquées contre le coussin et la vessie. L'introduction de produit de contraste dans le récipient induit une augmentation de son volume qui tend à appliquer une force de poussée sur le coussin et la vessie. Cette force induit l'expulsion de fluide hydraulique hors de la vessie. Plus précisément, lorsque les parois du récipient sont plaquées contre la vessie et le coussin, l'introduction d'une quantité donnée de produit de contraste à l'intérieur du récipient induit l'expulsion de la même quantité de fluide hydraulique hors de la vessie. Ainsi, une fois les parois du récipient plaquées contre la vessie et le coussin, il est possible de contrôler avec précision la quantité de produit de contraste introduit dans le récipient en mesurant la quantité de fluide hydraulique expulsé de la vessie.

Le fait que le seuil d'ouverture de la valve à seuil soit supérieur à 1,5 bar (et de préférence supérieur ou égale à 2 bars) permet de garantir un bon plaquage du récipient 3 sur la vessie et le coussin avant même le moindre écoulement de produit de contraste hors du récipient, l'écoulement de produit de contraste hors du récipient requiérant un minimum de pression de 1,5 bar dans le récipient.

### Ligne de remplissage

La ligne de remplissage 2 comprend une tubulure 21 en amont de l'interface de couplage 26 et une tubulure 23 en aval de l'interface de couplage 26.

Dans une variante de réalisation, la longueur de la tubulure 21 en amont de l'interface de couplage 26 est comprise entre 400 et 800 millimètres afin de limiter les pertes de charge entre l'interface de couplage 26 et la source d'alimentation 4 lors de l'aspiration du fluide pour le remplissage du récipient 3.

Par ailleurs, le diamètre intérieur de la tubulure 21 en amont de l'interface de couplage 26 peut être supérieur au diamètre intérieur de la partie tubulaire aval 11 de la ligne d'injection 1. En particulier, le diamètre intérieur de la tubulure 21 en amont de l'unité de pompage 22 peut être compris entre 3 et 6 millimètres.

Le fait de maximiser le diamètre intérieur de la tubulure 21 en amont de l'interface de couplage 26 permet de limiter les pertes de charge entre la source d'alimentation primaire 4 et l'interface de couplage 26 lors de l'aspiration du fluide pour le remplissage du récipient 3.

De plus, la dureté de la tubulure 21 en amont de l'interface de couplage 26 peut être supérieure à la dureté de la tubulure 23 en aval de l'interface de couplage 26. En particulier, la dureté de la tubulure 21 en amont de l'interface de couplage 26 peut être égale ou supérieure à 70 ShA. Le fait de disposer d'une tubulure 21 de dureté égale ou supérieure à 70 ShA permet d'éviter un collabage de celle-ci lors de l'aspiration du fluide pour le remplissage du récipient 3. De préférence, la dureté de la tubulure est choisie inférieure à 90 ShA pour faciliter son enroulement lors de l'emballage et faciliter sa manutention par l'opérateur lors de l'installation sur l'injecteur.

Avantageusement, la dureté de la tubulure 23 en aval de l'interface de couplage 26 peut être choisie inférieure à la dureté de la partie tubulaire aval 11 de la ligne d'injection 1. Notamment, la dureté shore A de la tubulure 23 en aval de l'interface de couplage 26 peut être comprise entre 60 et 80 ShA.

Le fait que la dureté de la tubulure 23 soit inférieure ou égale à 80 ShA permet à la ligne de remplissage 2 d'absorber les pulsations éventuellement générées par l'unité de pompage 22 afin de limiter les risques de surpression dans l'ensemble à usage médical, surpression pouvant induire une fuite de produit vers le patient au travers du clapet anti-retour 13 à haut seuil de la ligne d'injection 1. Le fait que la dureté de la tubulure 23 soit supérieure ou égale à 60 ShA permet de limiter le risque de rupture de cette tubulure 23 sous l'effet de la pression.

En variante, le diamètre de la tubulure 23 en aval de l'interface de couplage 26 peut être choisi supérieur au diamètre de la partie tubulaire aval 11 de la ligne d'injection 1. Notamment, le diamètre de la tubulure 23 en aval de l'interface de couplage 26 peut être compris entre 3 et 6 millimètres. Là encore, ceci permet d'absorber les pulsations générées par l'unité de pompage 22.

La ligne de remplissage 2 peut aussi comprendre un clapet anti-retour 25 permettant le passage de liquide dans un unique sens, à savoir de la source d'alimentation primaire 4 vers le récipient 3. La présence d'un clapet anti-retour 25 permet d'isoler la ligne de remplissage 2 une fois la phase de remplissage terminée, et ceci sans nécessiter de manipulation de l'utilisateur préalablement à la mise en oeuvre de la phase d'administration du produit au patient.

La ligne de remplissage 2 peut comprendre un piégeur de bulle 24 - tel qu'une chambre compte-goutte - entre la source d'alimentation primaire 4 et le récipient 3. Ce piégeur de bulle 24 permet de chasser l'air entre la source d'alimentation primaire 4 et le récipient 3.

### Principe de fonctionnement de l'ensemble

Le principe de fonctionnement de l'ensemble à usage médical décrit ci-dessus est le suivant : lors de la mise en oeuvre d'une opération d'injection de produit à un patient, l'utilisateur sort l'ensemble à usage médical de son emballage. Il connecte :
- l'extrémité libre de la ligne de remplissage 2 à la source d'alimentation primaire 4, et
- l'extrémité libre de la ligne d'injection 1 au patient après avoir purgé celle-ci.

Lorsque le récipient 3 est intégré à l'ensemble, aucune étape de connexion de celui-ci n'est nécessaire, ce qui limite les risques de contamination de l'ensemble lors de sa mise en place.

L'utilisateur met ensuite en oeuvre la phase de remplissage. L'unité de pompage 22 est actionnée pour remplir le récipient 3 avec le produit à injecter. Le produit à injecter se déplace entre la source d'alimentation primaire 4 et le récipient 3. Le clapet anti-retour 13 à haut seuil de déclenchement empêche le passage du produit dans la partie tubulaire aval 11 de la ligne d'injection 1 afin d'éviter tout risque de sortie intempestive de produit à injecter vers l'extrémité libre de la ligne d'injection 1 lors de la phase de remplissage.

Le récipient 3 se remplit de produit à injecter. Lorsque la quantité de produit contenue dans le récipient 3 est suffisante, l'utilisateur interrompt la phase de remplissage pour mettre en oeuvre la phase d'administration du produit au patient en ayant préalablement purgé l'air contenu initialement dans les lignes de remplissage 2 et d'injection 1.

Le récipient 3 est alors mis sous pression - par exemple en utilisant un injecteur à poche ou un dispositif pousse seringue - pour induire le déplacement du produit entre le récipient 3 et le patient.

Le clapet anti-retour 25 de la ligne de remplissage 2 empêche le passage du produit entre le récipient 3 et la source d'alimentation primaire 4. Le produit traverse le conduit d'injection 15 et pénètre dans le patient. Lorsqu'une quantité de produit suffisante a été administrée au patient, la phase d'administration est interrompue soit automatiquement, soit par un actionnement de l'utilisateur.

Ainsi, l'ensemble décrit ci-dessus permet de réaliser des transferts rapides de produit liquide ou visqueux entre :
- une source d'alimentation primaire 4 et un récipient 3 d'une part, et
- le récipient 3 et un patient d'autre part,
tout en limitant les risques de contamination par l'utilisateur.

Le lecteur aura compris que de nombreuses modifications peuvent être apportées à l'ensemble à usage médical sans sortir matériellement des nouveaux enseignements et avantages décrits ici.

Par conséquent, toutes les modifications de ce type sont destinées à être incorporées à l'intérieur de la portée des revendications jointes.

## Revendications

1. Ensemble à usage médical pour l'injection à un patient d'un produit liquide ou visqueux, tel qu'un produit de contraste, **caractérisé en ce que** l'ensemble comporte :
- une ligne d'injection (1) pour le transfert du produit à injecter entre un récipient (3) et le patient, ladite ligne d'injection (1) comportant une partie tubulaire amont (12), une partie tubulaire aval (11) et un clapet anti-retour à seuil sur la partie tubulaire aval, ledit clapet anti-retour à seuil (13) permettant le passage du produit liquide depuis le récipient (3) vers le patient,
- une ligne de remplissage (2) pour le transfert du produit à injecter entre une source d'alimentation (4) et le récipient (3) par l'intermédiaire de la partie tubulaire amont (12), ladite ligne de remplissage (2) étant raccordée à la ligne d'injection (1) entre les parties tubulaires amont et aval (11, 12) et comprenant un clapet anti-retour (25) permettant le passage du produit liquide depuis la source d'alimentation (4) vers le récipient (3),
- une interface de couplage (26) sur la ligne de remplissage (2) destinée à être couplée à une unité de pompage (22) permettant le déplacement du liquide entre la source d'alimentation (4) et le récipient (3).

2. Ensemble à usage médical selon la revendication précédente, dans lequel la ligne de remplissage (2) comprend une tubulure (21) en amont de l'interface de couplage (26) et une tubulure (23) en aval de l'interface de couplage (26), la dureté de la tubulure (21) en amont de l'interface de couplage (26) étant supérieure à la dureté de la tubulure (23) en aval de l'interface de couplage (26).

3. Ensemble à usage médical selon la revendication précédente, dans lequel la dureté de la tubulure (21) en amont de l'interface de couplage (26) est égale ou supérieure à 70 ShA.

4. Ensemble à usage médical selon l'une des revendications précédentes, dans lequel la ligne de remplissage (2) comprend une tubulure (21) en amont de l'interface de couplage (26) et une tubulure (23) en aval de l'interface de couplage (26), le diamètre intérieur de la tubulure en amont (21) de l'interface de couplage (26) étant supérieur au diamètre intérieur la partie tubulaire aval (11) de la ligne d'injection (1).

5. Ensemble à usage médical selon la revendication précédente, dans lequel le diamètre intérieur de la tubulure (21) en amont de l'interface de couplage (26) est compris entre 3 et 6 millimètres.

6. Ensemble à usage médical selon l'une des revendications précédentes, dans lequel la ligne de remplissage (2) comprend une tubulure (21) en amont de l'interface de couplage (26) et une tubulure (23) en aval de l'interface de couplage (26), la dureté de la tubulure (23) en aval de l'unité de pompage (22) étant inférieure à la dureté de la partie tubulaire aval (11) de la ligne d'injection (1).

7. Ensemble à usage médical selon la revendication précédente, dans lequel la dureté shore de la tubulure (23) en aval de l'unité de pompage (22) est comprise entre 60 et 80 ShA.

8. Ensemble à usage médical selon l'une des revendications précédentes, dans lequel la ligne de remplissage (2) comprend une tubulure (21) en amont de l'interface de couplage (26) et une tubulure (23) en aval de l'interface de couplage (26), le diamètre de la tubulure (23) en aval de l'interface de couplage (26) étant supérieur au diamètre de la partie tubulaire aval (11) de la ligne d'injection (1).

9. Ensemble à usage médical selon l'une des revendications précédentes, lequel comprend en outre le récipient (3), ledit récipient (3) étant connecté à la ligne d'injection (1) préalablement à son utilisation.

10. Ensemble à usage médical selon la revendication précédente, dans lequel le récipient (3) est une poche composée de deux feuilles de matériau superposées et soudées à leur périphérie pour définir un espace destiné à contenir le produit liquide ou visqueux.

11. Ensemble à usage médical selon l'une des revendications précédentes, lequel comprend en outre une connexion inamovible (31) entre le récipient (3) et la ligne d'injection (1).

## Patentansprüche

1. Anordnung zur medizinischen Verwendung zur Injektion eines flüssigen oder viskosen Produkts, wie eines Kontrastmittels, in einen Patienten, **dadurch gekennzeichnet, dass** die Anordnung umfasst:
- eine Injektionsleitung (1) für den Transfer des zu injizierenden Produkts zwischen einem Behälter (3) und dem Patienten, wobei die Injektionsleitung (1) einen stromaufwärtigen rohrförmigen Abschnitt (12), einen stromabwärtigen rohrförmigen Abschnitt (11) und ein Schwellen-Rückschlagventil an dem stromabwärtigen rohrförmigen Abschnitt umfasst, wobei das Schwellen-Rückschlagventil (13) den Durchgang des flüssigen Produkts aus dem Behälter (3) zum Patienten gestattet,
- eine Füllleitung (2) für den Transfer des zu injizierenden Produkts zwischen einer Versorgungsquelle (4) und dem Behälter (3) mit Hilfe des stromaufwärtigen rohrförmigen Abschnitts (12), wobei die Füllleitung (2) mit der Injektionsleitung (1) zwischen dem stromaufwärtigen und dem stromabwärtigen rohrförmigen Abschnitt (11, 12) verbunden ist und ein Rückschlagventil (25) umfasst, das den Durchgang des flüssigen Produkts aus der Versorgungsquelle (4) zum Behälter (3) gestattet,
- eine Kupplungsschnittstelle (26) an der Füllleitung (2), die dazu bestimmt ist, mit einer Pumpeinheit (22) gekuppelt zu werden, wobei die Pumpeinheit, die Verschiebung der Flüssigkeit zwischen der Versorgungsquelle (4) und dem Behälter (3) gestattet.

2. Anordnung zur medizinischen Verwendung nach dem vorhergehenden Anspruch,
wobei die Fülleitung (2) einen Schlauch (21) stromaufwärts von der Kupplungsschnittstelle (26) und einen Schlauch (23) stromabwärts von der Kupplungsschnittstelle (26) umfasst, wobei die Härte des Schlauchs (21) stromaufwärts von der Kupplungsschnittstelle (26) größer ist als die Härte des Schlauchs (23) stromabwärts von der Kupplungsschnittstelle (26).

3. Anordnung zur medizinischen Verwendung nach dem vorhergehenden Anspruch,
wobei die Härte des Schlauchs (21) stromaufwärts von der Kupplungsschnittstelle (26) größer oder gleich 70 ShA ist.

4. Anordnung zur medizinischen Verwendung nach einem der vorhergehenden Ansprüche,
wobei die Füllleitung (2) einen Schlauch (21) stromaufwärts von der Kupplungsschnittstelle (26) und einen Schlauch (23) stromabwärts von der Kupplungsschnittstelle (26) umfasst, wobei der Innendurchmesser des Schlauchs (21) stromaufwärts von der Kupplungsschnittstelle (26) größer ist als der Innendurchmesser des stromabwärtigen rohrförmigen Abschnitts (11) der Injektionsleitung (1) .

5. Anordnung zur medizinischen Verwendung nach dem vorhergehenden Anspruch,
wobei der Innendurchmesser des Schlauchs (21) stromaufwärts von der Kupplungsschnittstelle (26) zwischen 3 und 6 Millimeter beträgt.

6. Anordnung zur medizinischen Verwendung nach einem der vorhergehenden Ansprüche,
wobei die Füllleitung (2) einen Schlauch (21) stromaufwärts von der Kupplungsschnittstelle (26) und einen Schlauch (23) stromabwärts von der Kupplungsschnittstelle (26) umfasst, wobei die Härte des Schlauchs (23) stromabwärts von der Pumpeinheit (22) kleiner ist als die Härte des stromabwärtigen rohrförmigen Abschnitts (11) der Injektionsleitung (1).

7. Anordnung zur medizinischen Verwendung nach dem vorhergehenden Anspruch,
wobei die Shore-Härte des Schlauchs (23) stromabwärts von der Pumpeinheit (22) zwischen 60 und 80 ShA beträgt.

8. Anordnung zur medizinischen Verwendung nach einem der vorhergehenden Ansprüche,
wobei die Füllleitung (2) einen Schlauch (21) stromaufwärts von der Kupplungsschnittstelle (26) und einen Schlauch (23) stromabwärts von der Kupplungsschnittstelle (26) umfasst, wobei der Durchmesser des Schlauchs (23) stromabwärts von der Kupplungsschnittstelle (26) größer ist als der Durchmesser des stromabwärtigen rohrförmigen Abschnitts (11) der Injektionsleitung (1).

9. Anordnung zur medizinischen Verwendung nach einem der vorhergehenden Ansprüche,
welche außerdem den Behälter (3) umfasst, wobei der Behälter (3) mit der Injektionsleitung (1) vor seiner Verwendung verbunden wird.

10. Anordnung zur medizinischen Verwendung nach dem vorhergehenden Anspruch,
wobei der Behälter (3) ein Beutel ist, der aus zwei Materialschichten besteht, die übereinandergelegt und an ihrem Umfang verschweißt sind, um einen Raum zu definieren, der dazu bestimmt ist, das flüssige oder viskose Produkt zu enthalten.

11. Anordnung zur medizinischen Verwendung nach einem der vorhergehenden Ansprüche,
welche außerdem eine nicht-lösbare Verbindung (31) zwischen dem Behälter (3) und der Injektionsleitung (1) umfasst.

## Claims

1. Assembly for medical use for injecting a patient with a liquid or viscous product, such as a contrast product, **characterized in that** the assembly comprises:
- an injection line (1) for the transfer of the product to be injected between a container (3) and the patient, said injection line (1) comprising an upstream tubular part (12), a downstream tubular part (11) and a thresholded non-return valve on the downstream tubular part, said thresholded non-return value (13) allowing the liquid product to pass from the container (3) to the patient,
- a filling line (2) for the transfer of the product to be injected between a feed source (4) and the container (3) via the upstream tubular part (12), said filling line (2) being connected to the injection line (1) between the upstream and downstream tubular parts (11, 12) and comprising a non-return valve (25) allowing the liquid product to pass from the feed source (4) to the container (3),
- a coupling interface (26) on the filling line (2) intended to be coupled to a pumping unit (22) allowing for the displacement of the liquid between the feed source (4) and the container (3).

2. Assembly for medical use according to the preceding claim, in which the filling line (2) comprises a nozzle (21) upstream of the coupling interface (26) and a nozzle (23) downstream of the coupling interface (26), the hardness of the nozzle (21) upstream of the coupling interface (26) being greater than the hardness of the nozzle (23) downstream of the coupling interface (26).

3. Assembly for medical use according to the preceding claim, in which the hardness of the nozzle (21) upstream of the coupling interface (26) is equal to or greater than 70 ShA.

4. Assembly for medical use according to the one of the preceding claims, in which the filling line (2) comprises a nozzle (21) upstream of the coupling interface (26) and a nozzle (23) downstream of the coupling interface (26), the internal diameter of the nozzle (21) upstream of the coupling interface (26) being greater than the internal diameter of the downstream tubular part (11) of the injection line (1).

5. Assembly for medical use according to the preceding claim, in which the internal diameter of the nozzle (21) upstream of the coupling interface (26) is between 3 and 6 millimeters.

6. Assembly for medical use according to one of the preceding claims, in which the filling line (2) comprises a nozzle (21) upstream of the coupling interface (26) and a nozzle (23) downstream of the coupling interface (26), the hardness of the nozzle (23) downstream of the pumping unit (22) being less than the hardness of the downstream tubular part (11) of the injection line (1).

7. Assembly for medical use according to the preceding claim, in which the Shore hardness of the nozzle (23) downstream of the pumping unit (22) is between 60 and 80 ShA.

8. Assembly for medical use according to one of the preceding claims, in which the filling line (2) comprises a nozzle (21) upstream of the coupling interface (26) and a nozzle (23) downstream of the coupling interface (26), the diameter of the nozzle (23) downstream of the coupling interface (26) being greater than the diameter of the downstream tubular part (11) of the injection line (1) .

9. Assembly for medical use according to one of the preceding claims, which further comprises the container (3), said container (3) being connected to the injection line (1) prior to its use.

10. Assembly for medical use according to the preceding claim, in which the container (3) is a pocket made up of two sheets of material superposed and welded at their periphery to define a space intended to contain the liquid or viscous product.

11. Assembly for medical use according to one of the preceding claims, which further comprises a non-removable connection (31) between the container (3) and the injection line (1).
